# FASCICULE DE BREVET EUROPEEN

(11) **EP 0 642 775 B1**
(45) Date de publication et mention de la délivrance du brevet: **12.01.2000**
(21) Numéro de dépôt: 94402024.7
(22) Date de dépôt: 12.09.1994
(51) Int. Cl.: A61F 2/44

(54) **Prothèse de disque intervertébral**
Bandscheibenprothese
Intervertebral disc prosthesis

(30) Priorité: 14.09.1993 FR 9310917
(43) Date de publication de la demande: 15.03.1995
(73) Titulaire: COMMISSARIAT A L'ENERGIE ATOMIQUE, 75015 Paris (FR); UNIVERSITE PIERRE ET MARIE CURIE PARIS VI, 75252 Paris Cédex 05 (FR)
(72) Inventeur: Bainville, Daniel, F-37550 St Avertin (FR); Laval, François, F-37260 Monts (FR); Roy-Camille, Raymond, F-75007 Paris (FR); Saillant, Gérard, F-78170 La Celle St Cloud (FR); Lavaste, François, F-91240 St Michel sur Orge (FR)
(74) Mandataire: Dubois-Chabert, Guy

(56) Documents cités:
- EP-A- 0 277 282
- EP-A- 0 346 269
- EP-A- 0 356 112
- DE-A- 2 263 842
- DE-U- 9 000 094
- US-A- 4 874 389

## Description

### Domaine Technique

La présente invention a pour objet une prothèse destinée à remplacer un disque intervertébral endommagé de la colonne vertébrale.

Elle trouve une application dans le domaine de la chirurgie orthopédique.

### Etat de la technique

La colonne vertébrale est constituée par un ensemble de vertèbres superposées reliées les unes aux autres par des disques fibro-cartilagineux, appelés disques intervertébraux. Ces disques intervertébraux ont un rôle fondamental dans la statique et la dynamique de la colonne vertébrale : ils assurent la mobilité des vertèbres entre elles.

Ces disques intervertébraux sont souvent l'objet de désordres relatifs à un tassement de vertèbres, une hernie discale, un déplacement de vertèbres ou encore une dégénérescence arthrosique intervertébrale. Ces désordres sont, bien souvent, la cause de douleurs ou de gènes fonctionnelles rebelles aux traitements médicaux ; dans certains cas, ils peuvent même être invalidants.

Le procédé utilisé pour soulager les patients atteints de ces désordres consiste, généralement, en une intervention chirurgicale. Plusieurs techniques d'intervention chirurgicale sont actuellement connues.

La première technique consiste en une excision discale simple ; la zone endommagée du disque intervertébral est simplement supprimée, ce qui supprime de ce fait la biomécanique normale du disque. Les deux vertèbres adjacentes à ce disque ont alors une mobilité de l'une par rapport à l'autre, réduite. En outre, une telle excision ne constitue pas un remède définitif au mal du patient, la dégradation du disque pouvant continuer à évoluer.

La seconde technique d'intervention chirurgicale consiste en une arthrodèse intervertébrale par laquelle les deux vertèbres adjacentes au disque endommagé sont fusionnées. Ce procédé est, en effet, un remède définitif qui bloque l'évolution arthrosique, c'est-à-dire la dégradation, dans le disque. Ce procédé a pour principal inconvénient de supprimer toute mobilité discale.

La troisième technique, la plus répandue actuellement, consiste en une chirurgie arthroplastique intervertébrale de remplacement du disque endommagé par une prothèse. De nombreux types de prothèses discales sont connus et décrits dans l'ouvrage intitulé "The Artificial Disc", édité par Mario BROCK, H. Mickael MAYER et Klaus WEIGEL chez SPRINGER-VERLAG, 1991.

Selon cet ouvrage, les prothèses discales connues peuvent être classées en deux grandes catégories :
- les prothèses pour lesquelles la partie fibreuse du disque naturel est conservée et utilisée comme enveloppe de prothèse. Le noyau intérieur du disque naturel est alors remplacé, soit par un produit polymérisant injecté dans l'enveloppe, soit par des pièces élastiques introduites dans l'enveloppe, soit par un sac inséré dans l'enveloppe et gonflé au moyen d'un liquide ou d'un polymère. Une telle prothèse est décrite, notamment, dans les demandes de brevet US-A-4 772 287 et EP-A-0 277 282. La difficulté principale, pour réaliser une telle prothèse, réside dans le fait qu'il faille retrouver la géométrie et la résistance en compression du disque naturel. En outre, une telle prothèse ne peut être considérée comme un remède définitif pour le patient, la partie fibreuse conservée du disque pouvant continuer à se dégrader ;
- les prothèses entièrement artificielles qui se substituent à un disque naturel complètement retiré.

Une première technique de réalisation de ces prothèses entièrement artificielles consiste à introduire une articulation glissante entre les deux vertèbres. Cette articulation peut être en contact direct sur les plateaux vertébraux ; elle peut aussi consister en une rotule centrée comme le décrit le document FR-A-2 372 622. Une telle prothèse à rotule centrée a pour inconvénient que le déplacement d'une vertèbre par rapport à une autre entraîne un frottement au niveau de la rotule. Ces frottements peuvent produire, relativement rapidement, une irritation et, après un certain temps, des débris d'usure migrants. Ces débris d'usure peuvent provoquer une réaction à corps étranger qui, si elle se développe au contact du canal médulaire, peut être la cause de troubles neurologiques.

La seconde technique de réalisation de prothèses entièrement artificielles consiste à utiliser une architecture déformable reliée, rigidement, aux plateaux vertébraux adjacents. Une telle prothèse est décrite, par exemple, dans le brevet FR-A-2 124 815. Cette prothèse consiste, plus précisément, en un élément affectant la forme d'un disque intervertébral et réalisé dans un matériau élastomère du type silicone. Elle a pour inconvénient de ne pouvoir assurer qu'une mobilité limitée des vertèbres adjacentes, son aptitude à se déformer étant relativement limitée. En outre, cette prothèse n'interdit pas l'expansion radiale du matériau élastomère lors des contraintes exercées sur la prothèse, ce qui se traduit par un risque d'expulsion vers le canal médullaire.

Le document EP-A-0 346 269 décrit une prothèse de disque intervertébral comportant des moyens de renforcement à la périphérie de la prothèse.

Le document DE-A-2 263 842 décrit une prothèse de disque intervertébral comportant deux flasques entre lesquels est inséré un noyau central entouré d'un anneau en matériau synthétique. Le noyau central se meut entre les deux flasques telle une rotule, c'est-à-dire qu'il évolue avec glissement.

Une telle prothèse n'autorise donc qu'un mouvement de translation axiale par compression relativement limité, surtout dans les modes de réalisation des figures 7, 8 et 9 ; un tel mouvement est nécessaire pour permettre un bon amortissement des chocs et des efforts. En outre, le mouvement de translation axiale par traction permis par cette prothèse peut être néfaste, puisque les contacts glissants autorisent la séparation des pièces, ce qui peut produire un déboîtement de la prothèse. De plus, cette prothèse n'assure qu'une rotation limitée autour des trois axes principaux du trièdre de référence à cause du contact entre les bordures des vertèbres adjacentes.

Un autre exemple de ce type de prothèse est décrit dans le brevet EP-A-0 356 112. Schématiquement, cette prothèse comporte plusieurs couches concentriques d'élastomères d'élasticités différentes, reliées par adhésion à deux plaques métalliques ou non. Ce montage est prévu dans deux dimensionnements, relativement au disque que la prothèse doit remplacer : 100 et environ 80 %. Elle présente le même problème d'expansion radiale des élastomères, quasi-incompressibles en volume, sous contrainte ; de plus la seule utilisation de l'adhésion ne présente pas une assurance de maintien à très long terme, in vivo.

### Exposé de l'invention

La présente invention a justement pour but de remédier à ces inconvénients en proposant une prothèse discale comportant deux demi-enveloppes fixées rigidement sur les vertèbres et reliées au moyen d'un coussin compressible apte à absorber les déformations.

De façon plus précise, l'invention concerne une prothèse préformée destinée à remplacer un disque intervertébral détérioré sans frottement ni glissement. Cette prothèse comporte :
- deux demi-enveloppes rigides en forme de coupelles, fixées chacune solidairement sur une des deux vertèbres adjacentes au disque intervertébral à remplacer ;
- des moyens flexibles disposés entre les deux demi-enveloppes et comportant :
   - un coussin de compression absorbant des déformations consécutives à un déplacement de l'une des vertèbres adjacentes par rapport à l'autre et comprenant au moins une couche centrale et une couche extérieure (C1, C2) concentriques constituées de matériaux de raideurs différentes, la couche centrale étant déformable et compressible en volume de façon à absorber élastiquement les déformations de la couche extérieure qui est déformable et peu compressible en volume ; et
   - un système anti-expulsion bordant le coussin de compression, pour empêcher toute expansion de la couche extérieure hors du volume défini par les deux demi-enveloppes, les déformations de la couche extérieure peu compressible en volume étant absorbées par la couche centrale compressible en volume.

Avantageusement, les matériaux constituant le coussin de compression sont fixés aux demi-enveloppes par adhésion et au moyen de nervures radiales, sensiblement circulaires.

Selon l'invention, les couches des matériaux constituant le coussin de compression sont concentriques.

La prothèse selon l'invention comporte une membrane fixée sur chacune des demi-enveloppes et entourant le coussin de compression pour assurer une étanchéité entre ledit coussin de compression et l'environnement.

De façon avantageuse, le système anti-expulsion comporte des moyens annulaires de mise en forme du coussin de compression pour limiter l'expansion dudit coussin de compression hors du volume défini par les deux demi-enveloppes.

Selon un premier mode de réalisation, ces moyens annulaires de mise en forme comportent une pluralité d'anneaux métalliques de section circulaire et de forme adaptée au contour du coussin de compression, maintenus ensemble par un moyen de maillage, deux de ces anneaux métalliques étant sertis en bordure des deux demi-enveloppes de façon à assurer un écart maximum entre lesdites demi-enveloppes.

Selon un second mode de réalisation, ces moyens annulaires de mise en forme comportent une lame métallique de forme adaptée au contour du coussin de compression, deux anneaux métalliques de section circulaire sertis en bordure des deux demi-enveloppes et un moyen de maillage reliant la lame aux anneaux et assurant un écart maximum entre lesdites demi-enveloppes.

Selon un troisième mode de réalisation, les moyens annulaires de mise en forme comportent un fil enroulé en hélice autour du coussin de compression.

### Brève description des dessins

- La figure 1 représente une vue générale en perspective d'une portion de colonne vertébrale incluant une prothèse discale selon l'invention ;
- La figure 2 représente, selon une vue en coupe, un mode de réalisation de la prothèse discale de l'invention ; et
- La figure 3 représente, selon une vue en coupe, un autre mode de réalisation de la prothèse discale de l'invention.

### Exposé détaillé des modes de réalisation

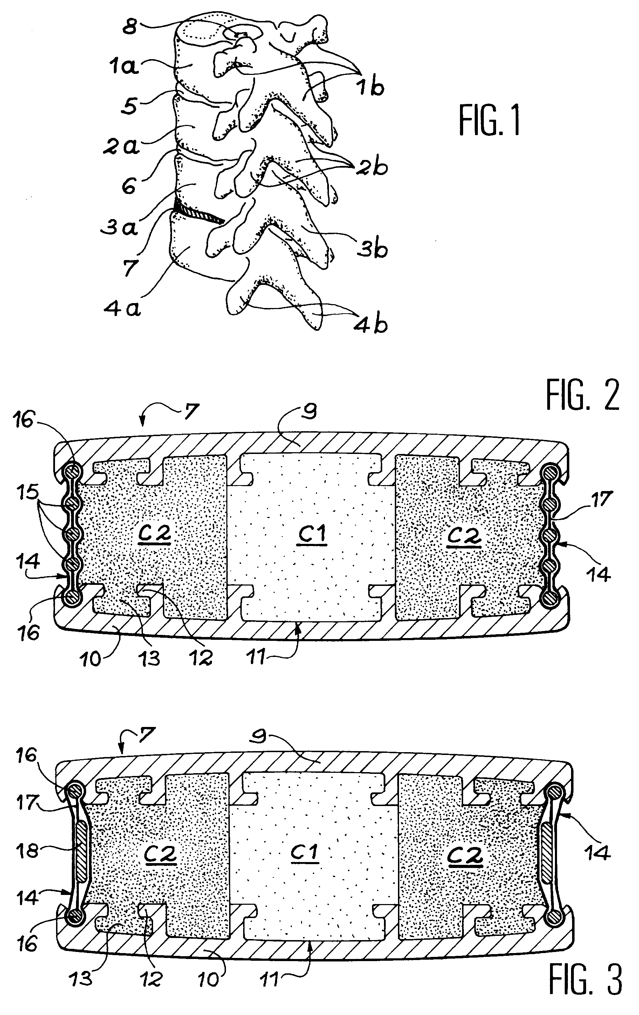

Sur la figure 1, on a représenté une portion de colonne vertébrale comportant quatre vertèbres 1, 2, 3, 4 reliées les unes aux autres par des disques intervertébraux. Selon l'exemple particulier représenté sur cette figure 1, les vertèbres 1 et 2 et les vertèbres 2 et 3 sont reliées, respectivement, par les disques intervertébraux 5 et 6. Selon cet exemple, les vertèbres 3 et 4 sont reliées par une prothèse discale 7, qui est représentée en hachures sur la figure 1.

On reconnait sur cette figure, le trou vertébral 8 que remplit la moëlle spinale. On reconnaît, en outre, sur chaque vertèbre 1, 2, 3, 4, le corps vertébral, respectivement 1a, 2a, 3a, 4a ainsi que les apophyses 1b, 2b, 3b, 4b.

Tout comme les disques intervertébraux 5 et 6, la prothèse discale 7 a une forme extérieure de "haricot" et est disposée entre les corps vertébraux 3a et 4a des vertèbres 3 et 4.

Sur la figure 2, on a représenté une vue en coupe d'un premier mode de réalisation de la prothèse discale 7. Cette prothèse discale 7 comporte deux demi-enveloppes 9 et 10 en forme de coupelles, appelées aussi cupules 9 et 10. Ces cupules 9 et 10 sont métalliques et fixées chacune sur le corps vertébral de l'une des deux vertèbres adjacentes. Elles peuvent, par exemple, être réalisées en titane allié ou en alliage chrome-molybdène.

Selon l'exemple de la figure 1, la cupule 9 est fixée sur le corps 3a de la vertèbre 3 et la cupule 10 sur le corps 4a de la vertèbre 4. Chacune de ces cupules 9 et 10 est en contact, par sa paroi convexe, avec l'une des vertèbres adjacentes, les parois concaves des cupules 9 et 10 se faisant face. La prothèse discale 7 reproduit ainsi globalement la forme du disque intervertébral qu'elle remplace.

La fixation des cupules 9 et 10 métalliques sur les vertèbres adjacentes peut être réalisée par les procédés classiques de chirurgie orthopédique, à savoir la fixation par vissage ou par broches. Cette fixation peut également être réalisée par ciment, par réhabitation par les tissus de la surface spécialement traitée (hydroxyapatite) ou encore par une combinaison de ces procédés. Quel que soit le mode de réalisation de la liaison adopté, cette liaison est rigide et assure une solidarisation de chaque cupule 9 et 10 sur l'une des vertèbres adjacentes.

Entre ces cupules 9 et 10 sont introduits des moyens flexibles. Ces moyens flexibles comprennent un coussin de compression 11 réalisé en plusieurs couches de matériaux ayant des coefficients de compressibilité différents. Selon un mode de réalisation préféré de l'invention, ces matériaux sont des polymères aptes à adhérer à la paroi concave de chaque cupule.

Selon ce mode de réalisation préféré de l'invention, la paroi concave de chaque cupule 9 et 10 comporte des nervures 12 formant, entre, elles, des gorges 13 dans lesquelles pénètre en partie le coussin de compression 11. Ces nervures 12 assurent le maintien du coussin de compression dans le volume que définissent les deux cupules 9 et 10.

Des nervures radiales peuvent, en outre, permettre de limiter la rotation relative des cupules 9 et 10, par l'intermédiaire du coussin de compression 11.

En outre, les parois concaves des cupules 9 et 10 peuvent être préalablement soumises à des traitements aptes à augmenter la capacité d'adhérence des polymères du coussin 11 sur le métal des cupules 9 et 10.

Selon l'exemple particulier représenté sur la figure 2, le coussin 11 comporte deux couches de polymères qui peuvent être, soit moulées in-situ entre les deux cupules, soit rapportées entre les cupules. Ces polymères ont des coefficients de compressibilité et des élasticités différents les uns des autres (c'est-à-dire des raideurs différentes) et sont répartis de façon à obtenir la flexibilité désirée de la prothèse. De façon plus précise, cette flexibilité dépend :
- des caractéristiques mécaniques (module d'élasticité, compressibilité volumique, etc.) des polymères utilisés ; et
- de l'agencement de ces couches de polymères.

Selon le mode de réalisation représenté sur la figure 2, les couches de polymères sont agencées de façon à être concentriques, perpendiculaires au plan des cupules.

Comme le sait l'homme de l'art, la fonction du disque à remplacer varie suivant son emplacement dans la colonne vertébrale. Un disque de vertèbres lombaires a des fonctions autres qu'un disque de vertèbres dorsales ou de vertèbres cervicales. La prothèse discale selon l'invention reproduit donc ces différentes fonctions en faisant varier l'élasticité globale de la prothèse. Pour ce faire et en fonction de l'élasticité et de la compressibilité de chaque matériau, différents agencements des couches de polymère peuvent être réalisés :
- un agencement tel que le module d'élasticité des polymères soit croissant ou décroissant du centre de la prothèse vers l'extérieur l'un des matériaux étant de plus compressible en volume ; et
- un agencement tel que le module d'élasticité des polymères soit alterné croissant/décroissant, s'il y a plus de deux couches, l'un des matériaux étant de plus compressible en volume.

Selon l'exemple représenté sur la figure 2, les couches de polymères du coussin 11 sont disposées de façon concentrique les unes aux autres. La couche "noyau" C1, qui est la couche la plus centrale du coussin 11, est réalisée en un polymère compressible en volume et donc apte à absorber les déformations subies par les couches voisines.

La couche extérieure C2, c'est-à-dire la couche adjacente à la couche noyau C1, est réalisée en un polymère élastique, mais quasi-incompressible en volume.

Selon l'invention, une membrane 14 entoure le coussin de compression 11 sur sa périphérie, réalisant ainsi une barrière étanche apte à empêcher toute migration de particules des matériaux compressibles du coussin 11 vers l'extérieur de la prothèse. Selon l'exemple de la figure 2, cette membrane 14 est concentrique aux couches C1, C2.

Selon un mode de réalisation, cette membrane 14 est un film de polyéthylène.

Cette membrane peut, en outre, affecter la forme d'un soufflet ; elle permet ainsi d'améliorer la résistance en fatigue lors des mouvements de flexion.

En outre, la membrane 14 est fixée de part et d'autre aux cupules 9 et 10 pour assurer l'étanchéité du coussin. Une telle étanchéité du coussin 11, et donc de la prothèse, permet de limiter le transfert, vers l'organisme, de particules résultant de la fatigue des matériaux du coussin 11. Ceci a pour conséquence d'assurer une meilleure tolérance de la prothèse par l'organisme et donc une plus longue durée d'implantation.

Selon l'invention, les moyens flexibles comportent en outre des moyens annulaires de mise en forme du coussin 11. Ces moyens annulaires sont rigides radialement et assurent une limite de l'expansion radiale des matériaux du coussin 11 hors du volume défini par les cupules 9 et 10. Ces moyens annulaires assurent une géométrie identique de la prothèse, quelles que soient les déformations subies par le coussin 11. Ces moyens annulaires constituent, de ce fait, un système anti-expulsion vis-à-vis du canal médullaire.

Selon le mode de réalisation représenté sur la figure 2, ces moyens annulaires comportent une pluralité d'anneaux 15 métalliques, de section circulaire et de forme adaptée au contour du coussin de compression ; ils comportent, en outre, deux anneaux 16 métalliques disposés de part et d'autre de la pluralité d'anneaux 15 et en contact avec chacune des cupules 9 et 10. Ces anneaux 16 peuvent être, par exemple, sertis en bordure des cupules 9 et 10 de façon à assurer la fermeture de la membrane 14, autour du coussin 11.

Selon le mode de réalisation représenté sur la figure 2, un maillage 17 assure le maintien des anneaux 15 entre eux et le positionnement des anneaux 16 en bordure de cupules. Ce maillage 17 est disposé sensiblement perpendiculairement au plan des cupules 9 et 10 et sur toute la périphérie du coussin 11 .

Ce maillage peut être réalisé, par exemple, en nylon.

Selon un mode de réalisation de l'invention, la membrane 14, les anneaux 16 et le maillage 17 peuvent être intégrés : les anneaux 16 et le maillage 17 par tressage et la membrane 14 par surmoulage.

Sur la figure 3, on a représenté une vue en coupe d'un second mode de réalisation de la prothèse discale 7. Tout comme le mode de réalisation de prothèse discale montré sur la figure 2, la prothèse discale 7 représentée sur cette figure 3 comporte :
- deux cupules 9 et 10 comportant des nervures 12 formant des gorges 13 ;
- un coussin de compression 11 comportant plusieurs couches C1, C2 de polymères ayant des capacités de compression différentes ;
- une membrane 14 entourant le coussin 11.

Cette prothèse discale 7 comporte, de plus, des moyens annulaires de mise en forme du coussin 11. Selon ce second mode de réalisation, ces moyens annulaires consistent en une lame circulaire 18 métallique, de forme adaptée au contour du coussin 11 et reliée aux deux anneaux 16 au moyen du maillage 17. Cet ensemble lame 18/anneaux 16/maillage 17 a un rôle tout à fait identique aux moyens annulaires décrits dans le premier mode de réalisation de la prothèse (figure 2).

Selon un troisième mode de réalisation de la prothèse discale, les moyens annulaires de mise en forme du coussin consistent en un fil enroulé en hélice autour de ce coussin de compression. Ce mode de réalisation a pour avantage d'être facile à mettre en oeuvre. En outre, ces moyens annulaires permettent une stabilité du positionnement des spires entre elles, une limitation de la rotation relative des cupules et une contribution à la résistance en traction et compression de la prothèse.

On comprendra donc aisément qu'une telle prothèse discale permette des mouvements d'une vertèbre par rapport à sa vertèbre voisine par déformation du coussin de compression 11 tout en assurant une étanchéité de la prothèse vis-à-vis de l'organisme. Une telle prothèse permet donc de limiter les risques de rejet de la prothèse par l'organisme.

## Revendications

1. Prothèse préformée destinée à remplacer un disque intervertébral détérioré sans frottement ni glissement comportant :
- deux demi-enveloppes (9, 10) rigides en forme de coupelles, fixées chacune solidairement sur une des deux vertèbres (3, 4) adjacentes au disque intervertébral à remplacer ;
- des moyens flexibles disposés entre les deux demi-enveloppes et comportant :
• un coussin de compression absorbant des déformations consécutives à un déplacement de l'une des vertèbres adjacentes par rapport à l'autre et comprenant au moins une couche centrale et une couche extérieure (C1, C2) concentriques constituées de matériaux de raideurs différentes, la couche centrale étant déformable et compressible en volume de façon à absorber élastiquement les déformations de la couche extérieure qui est déformable et peu compressible en volume ; et
• un système anti-expulsion bordant le coussin de Compression, pour empêcher toute expansion de la couche extérieure hors du volume défini par les deux demi-enveloppes, les déformations de la couche extérieure peu compressible en volume étant absorbées par la couche centrale compressible en volume.

2. Prothèse selon la revendication 1, caractérisée en ce que les matériaux constituant le coussin de compression sont fixés aux demi-enveloppes par adhésion et au moyen de nervures.

3. Prothèse selon la revendication 1 ou 2, caractérisée en ce qu'elle comporte une membrane (14) fixée sur chacune des demi-enveloppes et entourant le coussin de compression pour en assurer son étanchéité.

4. Prothèse selon la revendication 1, caractérisée en ce que le système anti-expulsion comporte des moyens annulaires (14, 17) de mise en forme du coussin de compression.

5. Prothèse selon la revendication 4, caractérisé en ce que les moyens annulaires de mise en forme comportent une pluralité d'anneaux métalliques (15) de section circulaire et de forme adaptée au contour du coussin de compression, maintenus ensemble par un moyen de maillage (17), deux de ces anneaux métalliques (16) étant sertis en bordure des deux demi-enveloppes de façon à assurer un écart maximum entre lesdites demi-enveloppes.

6. Prothèse selon la revendication 4, caractérisée en ce que les moyens annulaires de mise en forme comportent une lame métallique (18) de forme adaptée au contour du coussin de compression, deux anneaux métalliques (16) de section circulaire sertis en bordure des deux demi-enveloppes et un moyen de maillage (17) reliant la lame aux anneaux et assurant un écart maximum entre lesdites demi-enveloppes.

7. Prothèse selon la revendication 4, caractérisée en ce que les moyens annulaires de mise en forme comportent un fil enroulé en hélice autour du coussin de compression.

## Claims

1. Preshaped prosthesis for replacing a deteriorated intervertebral disk without friction or sliding, comprising:
- two rigid half-envelopes (9, 10) shaped like cups and each integrally fixed to one of the two vertebrae (3, 4) adjacent to the intervertebral disk to be replaced,
- flexible means positioned between the two half-envelopes and incorporating:
a compression cushion absorbing deformations resulting from a displacement of one of the adjacent vertebrae with respect to the other and having at least one central layer and one outer layer (C1, C2), which are concentric and made from materials with different rigidities, the central layer being deformable and volume-compressible so as to elastically absorb the deformations of the outer layer, which is deformable and only slightly volume-compressible and
an anti-expulsion system bordering the compression cushion, to prevent any expansion of the outer layer out of the volume defined by the two half-envelopes, the deformations of the outer, slightly volume-compressible layer being absorbed by the central, volume-compressible layer.

2. Prosthesis according to claim 1, characterized in that the materials constituting the compression cushion are fixed to the half-envelopes by adhesion and ribs.

3. Prosthesis according to claim 1 or 2, characterized in that it incorporates a membrane (14) fixed to each of the half-envelopes and surrounding the compression cushion in order to ensure the sealing thereof.

4. Prosthesis according to claim 1, characterized in that the anti-expulsion system incorporates annular means (14, 17) for shaping the compression cushion.

5. Prosthesis according to claim 4, characterized in that the annular shaping means incorporate a plurality of metal rings (15) having a circular section and a shape adapted to the contour of the compression cushion and which are maintained together by a lattice means (17), two of the said metal rings (16) being crimped on the border of the two half-envelopes so as to ensure a maximum distance between said half-envelopes.

6. Prosthesis according to claim 4, characterized in that the annular shaping means incorporate a metal strip (18) having a shape adapted to the contour of the compression cushion, two metal rings (16) with a circular section and crimped on the border of the two half-envelopes and a lattice means (17) connecting the strip to the rings and ensuring a maximum distance between said half-envelopes.

7. Prosthesis according to claim 4, characterized in that the annular shaping means incorporate a wire wound helically around the compression cushion.

## Patentansprüche

1. Vorgeformte Prothese, dazu bestimmt eine beschädigte Bandscheibe zu ersetzen, gleit- und reibungsfrei, umfassend:
- flexible Einrichtungen, angeordnet zwischen den beiden Halbhüllen und enthaltend:
· ein Druckkissen, das Verformungen absorbiert, verursacht durch die Verschiebung bzw. Bewegung eines der benachbarten Wirbel in Bezug auf den anderen, und das wenigstens eine zentrale Schicht und eine äußere Schicht (C1, C2) umfasst, konzentrisch und gebildet aus Materialien mit unterschiedlichen Steifigkeiten, wobei die zentrale Schicht verformbar und im Volumen zusammendrückbar ist, um elastisch die Verformungen der äußeren Schicht zu absorbieren, die verformbar und im Volumen wenig zusammendrückbar ist; und
· ein das Druckkissen umgrenzendes Anti-Austreibsystem, um jede Ausdehnung der äußeren Schicht über das durch die beiden Halbhüllen definierte Volumen hinaus zu verhindern, wobei die Verformungen der im Volumen wenig zusammendrückbaren äußeren Schicht durch die im Volumen zusammendrückbare zentrale Schicht absorbiert werden.

2. Prothese nach Anspruch 1, dadurch gekennzeichnet, dass die das Druckkissen bildenden Materialien mittels Haftung und durch Rippen befestigt werden.

3. Prothese nach Anspruch 1 oder 2, dadurch gekennzeichnet, dass sie eine Membran (14) umfasst, die an jeder der Halbhüllen befestigt ist und das Druckkissen umgibt, um dessen Dichtheit sicherzustellen.

4. Prothese nach Anspruch 1, dadurch gekennzeichnet, dass das Anti-Austreibsystem ringartige Formungseinrichtungen (14, 17) des Druckkissens umfasst.

5. Prothese nach Anspruch 4, dadurch gekennzeichnet, dass die ringartigen Formungseinrichtungen eine Vielzahl von Metallringen (15) mit kreisrundem Querschnitt und einer der Kontur des Druckkissens angepassten Form umfassen, zusammengehalten durch eine Vernetzungseinrichtung (17), wobei zwei dieser Metallringe (16) eingefasst sind in den Rändern der beiden Halbhüllen, um einen maximalen Abstand zwischen den genannten Halbhüllen sicherzustellen.

6. Prothese nach Anspruch 4, dadurch gekennzeichnet, dass die ringartigen Formungseinrichtungen eine metallische Lamelle (18) umfassen, deren Form an die Kontur des Druckkissens angepasst ist, wobei zwei Metallringe (16) mit kreisrundem Querschnitt, eingefasst am Rand der beiden Halbhüllen, und eine Vernetzungseinrichtung (17) die Lamelle mit den Ringen verbinden und einen maximalen Abstand zwischen den genannten Halbhüllen sicherstellen.

7. Prothese nach Anspruch 4, dadurch gekennzeichnet, dass die ringartigen Formungseinrichtungen einen spiralförmig um das Druckkissen gewickelten Draht umfassen.
